# EUROPÄISCHE PATENTANMELDUNG

(11) **EP 2 014 658 A1**
(43) Veröffentlichungstag der Anmeldung: **14.01.2009**
(21) Anmeldenummer: 07011966.4
(22) Anmeldetag: 19.06.2007
(51) Int. Cl.: C07D 413/04

(54) **Nicotinamid-Derivate und Verfahren zu deren Herstellung**

(71) Anmelder: Bayer CropScience AG, 40789 Monheim (DE)
(72) Erfinder: Pazenok, Dr. Sergiy, 42699 Solingen (DE); Stelzer, Dr. Uwe, Kansas City MO 64120 (US); Blaschke, Harry, 42327 Wuppertal (DE); Neeff, Arnd, 58256 Ennepetal (DE); Mulder, Lubbertus, 58135 Hagen -Haspe (DE)

(57) **Zusammenfassung**

Beschreiben werden Nicotinamid-Verbindungen der allgemeinen Formel (1) 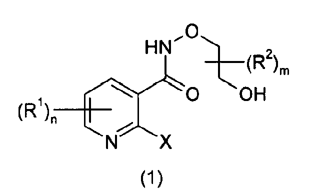 und Verfahren zu deren Herstellung.

## Beschreibung

Die Erfindung betrifft spezifische Nicotinamid-Verbindungen sowie Verfahren zu deren Herstellung.

Nicotinamid-Derivate sind wichtige Synthesebausteine zur Herstellung von agrochemischen Wirkstoffen, insbesondere zur Herstellung von Dioxazinderivaten, speziell von Dioxazin-Pyridinyl-Suflonylharnstoffen.

Entsprechende Dioxazin-Pyridinyl-Suflonylharnstoffe werden zum Beispiel in US 5,476,936 beschrieben. Die Synthese von solchen Verbindungen verläuft über die Umsetzung von Nicotinsäureestern mit Hydroxylamin und anschließende Umsetzung mit Dibromethan gemäß nachfolgender Reaktionsgleichung:

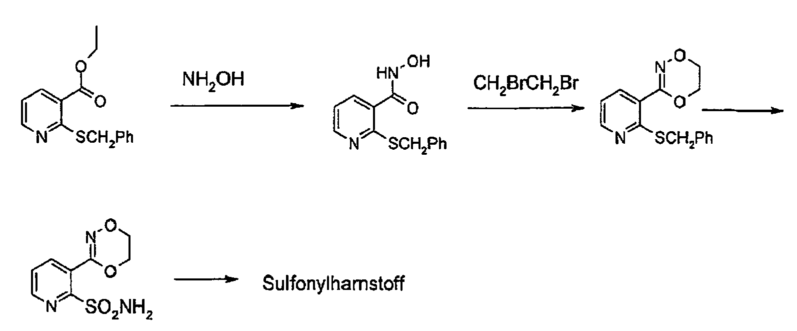

Die isolierte Ausbeute von 21 % und die Anwendung des hochtoxischen und umweltschädigenden Dibromethans macht die Realisierung eines solchen Verfahrens zum Aufbau von einem Dioxazin-Ring in entsprechenden Nicotinamid-Verbindungen (d.h. in einem mit einem Dioxazin-Ring substituiertem Pyridin) unattraktiv und teuer.

Daher besteht Bedarf nach einem alternativen Zugang zu einem Dioxazin-Ring, welcher kostengünstig und umweltschonend ist und die gewünschten Nicotinamid-Verbindungen mit guter Ausbeute und hoher Reinheit liefert.

In der zeitgleich eingereichten europäischen Patentanmeldung der Anmelderin (Bayer CropScience AG) mit dem Titel "Verfahren zur Herstellung von DioxazinDerivaten" wird ein kostengünstiger präparativer Zugang zu entsprechenden Nicotinamid-Verbindungen (d.h. mit einem Dioxazin-Ring substituierte Pyridine) beschrieben. Dieser präparative Zugang geht von einem Nicotinamid-Derivat der allgemeinen Formel (1) aus 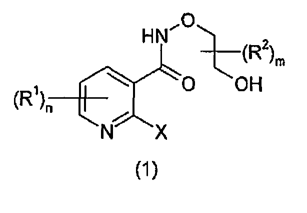 und ermöglicht die effiziente Herstellung entsprechender Nicotinamid-Verbindungen unter Verzicht auf die Verwendung von umweltschädigenden Substanzen wie Dibromethan mit hoher Ausbeute und Reinheit.

Um entsprechende Dioxazin-Ringe in Nicotinamid-Verbindungen gemäß diesem neuen Zugang aufzubauen, sind jedoch entsprechende Ausgangsverbindungen der allgemeinen Formel (1) erforderlich. Bisher gibt es keinen effizienten Zugang zu Verbindungen der allgemeinen Formel (1).

Die Aufgabe der vorliegenden Erfindung ist es daher, Nicotinamid-Derivate der allgemeinen Formel (1) bereitzustellen, welche in entsprechende Nicotinamid-Verbindungen umgewandelt werden können.

Darüber hinaus besteht die Aufgabe der vorliegenden Erfindung darin, Verfahren zur Herstellung von solchen Nicotinamid-Derivaten der allgemeinen Formel (1) zur Verfügung zu stellen, welche in entsprechende Nicotinamid-Verbindungen (d.h. mit einem Dioxazin-Ring substituierte Pyridine) umgewandelt werden können. Das Verfahren sollte dabei vorzugsweise mit guten Ausbeuten verlaufen und die gewünschten Zielverbindungen sollten vorzugsweise kostengünstig und vorzugsweise mit hoher Reinheit erhalten werden.

Die zuvor beschriebene Aufgabe wird zunächst durch Verbindungen der allgemeinen Formel (1) gelöst, 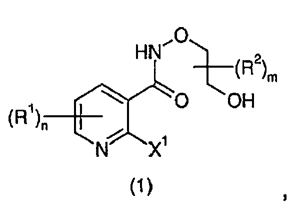 in welchen die Substituenten die folgende Bedeutung aufweisen:
X¹ Fluor, Chlor, Brom, Iod, SCN, oder S-R³, wobei
   R³ für Wasserstoff;
      gegebenenfalls substituiertes C₁-C₆-Alkyl;
      gegebenenfalls substituiertes C₃-C₆-Cycloalkyl;
      -(CH₂)ᵣ-C₆H₅ mit r = ganzzahlige Zahl von 0 bis 6, wobei der
      Alkylrest -(CH₂)ᵣ- gegebenenfalls substituiert sein kann; oder 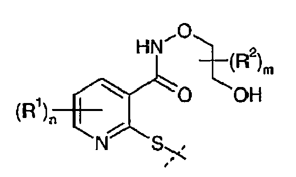 (d.h. Dimerstruktur der allgemeinen Formel (1)) steht;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n eine ganzzahlige Zahl von 0 bis 2;
R² unabhängig voneinander jeweils gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, wobei die Substituenten unabhängig voneinander ausgewählt sein können aus Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, (C₁-C₆-Alkoxy)carbonyl, (C₁-C₆-Alkyl)carbonyl oder C₃-C₆-Trialkylsilyl; und
m eine ganzzahlige Zahl von 0 bis 4.

In einer ersten bevorzugten Ausführungsform weisen die einzelnen Substituenten des Nicotin-Derivats der allgemeinen Formel (1) die folgende Bedeutung auf:
X¹ Chlor, S-R³, wobei
   R³ für gegebenenfalls substituiertes C₁-C₆-Alkyl; gegebenenfalls substituiertes C₃-C₆-Cycoalkyl; -(CH₂)ᵣ-C₆H₅ mit r = 1 bis 4 steht, wobei der Alkylrest -(CH₂)ᵣ- gegebenenfalls substituiert sein kann;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n 0 oder 1;
R² jeweils unabhängig voneinander für gegebenenfalls einfach oder mehrfach, gleich oder verschieden substituiertes C₁-C₄-Alkyl, C₃-C₆-Cycloalkyl, wobei die Substituenten unabhängig voneinander ausgewählt sein können aus Halogen, Cyano, Nitro, Hydroxy, C₁-C₄-Alkoxy, C₁-C₄-Haloalkoxy, C₁-C₄-Alkylthio, C₁-C₄-Alkylsulfinyl und C₁-C₄-Alkylsulfonyl; und
m eine ganzzahlige Zahl von 0 bis 2.

In einer zweiten, noch weiter bevorzugten Ausführungsform weisen die einzelnen Substituenten des Nicotin-Derivats der allgemeinen Formel (1) die folgende Bedeutung auf:
X¹ Chlor, S-R³, wobei
   R³ für gegebenenfalls substituiertes C₁-C₆-Alkyl; gegebenenfalls substituiertes C₃-C₆-Cycoalkyl; -(CH₂)ᵣ-C₆H₅ mit r = 1 oder 2 steht, wobei der Alkylrest -(CH₂)ₘ- gegebenenfalls substituiert sein kann;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n 0 oder 1;
R² gegebenenfalls einfach oder mehrfach, gleich oder verschieden substituiertes C₁-C₄-Alkyl, wobei die Substituenten unabhängig voneinander ausgewählt sein können aus Halogen, Cyano, Nitro, C₁-C₄-Alkoxy, C₁-C₄-Haloalkoxy; und
m 0 oder 1.

In einer dritten, noch weiter bevorzugten Ausführungsform weisen die einzelnen Substituenten des Nicotin-Derivats der allgemeinen Formel (1) die folgende Bedeutung auf:
X¹ S-CH₂-C₆H₅;
n 0; und
m 0.

Die zuvor beschriebene Aufgabe wird erfindungsgemäß darüber hinaus durch ein Verfahren zur Herstellung der zuvor beschriebenen Nicotinamid-Derivate der allgemeinen Formel (1) gelöst 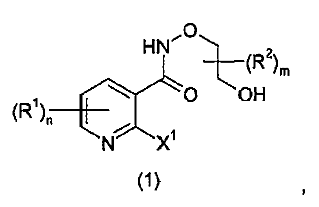 wobei die einzelnen Substituenten und Indizes die nachstehend definierten Bedeutungen aufweisen.

### Erste Ausführungsform

In einer ersten Ausführungsform ist das erfindungsgemäße Verfahren dadurch gekennzeichnet, dass zur Herstellung der gewünschten Nicotinamid-Derivate der allgemeinen Formel (1) Nicotinsäurechloride oder -ester der allgemeinen Formel (2) mit Aminoglykol der allgemeinen Formel (3) umgesetzt werden.

Die erfindungsgemäß vorgesehene Umsetzung von Nicoitinsäurechloriden oder -estern mit Aminoglykolen entspricht dabei der folgenden allgemeinen Reaktionsgleichung:

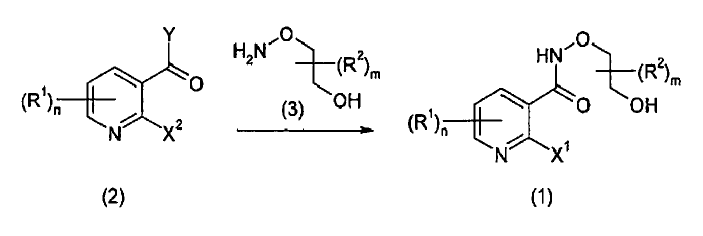

Im Folgenden werden nunmehr die Eduktverbindungen der allgemeinen Formeln (2) und (3) näher beschrieben.

In den erfindungsgemäß als Edukt vorgesehenen Nicoitinsäurechloriden oder -estern der allgemeinen Formel (2) 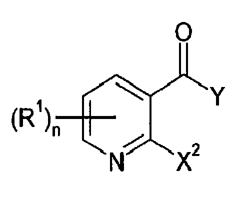 bedeutet
Y Chlor oder gegebenenfalls substituiertes -O(C₁-C₆-Alkyl);
X² Fluor, Chlor, Brom, Iod, SCN, oder S-R^{3'}, wobei
   R^{3'} für Wasserstoff;
      gegebenenfalls substituiertes C₁-C₆-Alkyl;
      gegebenenfalls substituiertes C₃-C₆-Cycoalkyl;
      -(CH₂)ᵣ-C₆H₅ mit r = 0 bis 6, wobei der Alkylrest -(CH₂)ᵣ-gegebenenfalls substituiert sein kann; oder 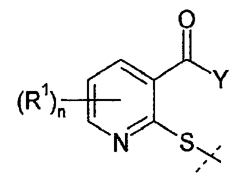 mit Y gleich Chlor oder gegebenenfalls substituiertes -O(C₁-C₆-Alkyl) (d.h. Dimerstruktur der allgemeinen Formel (2)) steht;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl-und Alkinylgruppen jeweils 2 bis 6 C-Atome, die Cycloalkylgruppen jeweils 3 bis 6 C-Atome und die Arylgruppen jeweils 6 oder 10 C-Atome enthalten können; und
n eine ganzzahlige Zahl von 0 bis 2.

Auch Salze der zuvor genannten Nicotinsäurechloride bzw. -ester der allgemeinen Formel (2) können verwendet werden.

Von den Nicotinsäurechloriden und -estern sind die entsprechenden Nicotinsäurechloride aufgrund ihrer höheren Reaktivität bevorzugt.

Als Nicoitinsäurechlorid insbesondere bevorzugte Verbindungen sind Verbindungen der allgemeinen Formel (2) und deren Salze, worin
Y für Chlor steht
X² für Chlor, S-R^{3'} steht, wobei
   R^{3'} für gegebenenfalls substituiertes C₁-C₆-Alkyl; gegebenenfalls substituiertes C₃-C₆-Cycoalkyl; -(CH₂)ᵣ-C₆H₅ mit r = 1 bis 4 steht, wobei der Alkylrest -(CH₂)ᵣ-gegebenenfalls substituiert sein kann;
R¹ für Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl-und Alkinylgruppen jeweils 2 bis 6 C-Atome, die Cycloalkylgruppen jeweils 3 bis 6 C-Atome und die Arylgruppen jeweils 6 oder 10 C-Atome enthalten können, steht; und
n für 0 oder 1 steht.

Als Nicoitinsäurechloride der allgemeinen Formel (2) besonders bevorzugt sind Verbindungen der Formel (2), worin
Y für Chlor steht;
X² für Chlor, S-CH₂-C₆H₅ steht; und
n für 0 steht.

Entsprechende Nicoitinsäurechloride der allgemeinen Formel (2) können ausgehend von den korrespondieren Nicoitincarbonsäuren durch Umsetzung mit einem Chlorierungsmittel wie Phosphoroxychlorid, Oxalylchlorid, Thionylchlorid, Phosgen, Phosphortrichlorid, oder Phosphorpentachlorid erhalten werden.

Entsprechende Nicoitinsäureester der allgemeinen Formel (2) können ausgehend von den korrespondieren Nicoitincarbonsäuren durch klassische Veresterung, beispielsweise durch Umsetzung mit Methanol, erhalten werden.

Die entsprechende Thiofunktionalisierung in ortho-Position zum Pyridin-Stickstoffatom in entsprechenden Nicotincarbonsäuren kann gemäß Vorschriften durchgeführt werden, welche in US 5,476,936 beschrieben sind.

In dem in der ersten Ausführungsform als Edukt vorgesehenem Aminoglykol der allgemeinen Formel (3) 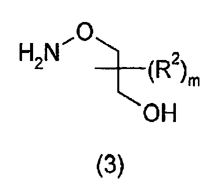 bedeutet
R² unabhängig voneinander jeweils gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, wobei die Substituenten unabhängig voneinander ausgewählt sein können aus Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, (C₁-C₆-Alkoxy)carbonyl, (C₁-C₆-Alkyl)carbonyl oder C₃-C₆-Trialkylsilyl; und
m eine ganzzahlige Zahl von 0 bis 3.

Als Aminoglykol der allgemeinen Formel (3) bevorzugte Verbindungen sind Verbindungen der allgemeinen Formel (3), worin
R² jeweils unabhängig voneinander für gegebenenfalls einfach oder mehrfach, gleich oder verschieden substituiertes C₁-C₄-Alkyl, C₃-C₆-Cycloalkyl steht, wobei die Substituenten unabhängig voneinander ausgewählt sein können aus Halogen, Cyano, Nitro, Hydroxy, C₁-C₄-Alkoxy, C₁-C₄-Haloalkoxy, C₁-C₄-Alkylthio, C₁-C₄-Alkylsulfinyl und C₁-C₄-Alkylsulfonyl; und
m für eine ganzzahlige Zahl von 0 bis 2 steht.

Als Aminoglykol der allgemeinen Formel (3) besonders bevorzugte Verbindungen sind Verbindungen der allgemeinen Formel (3), worin
R² für gegebenenfalls einfach oder mehrfach, gleich oder verschieden substituiertes C₁-C₄-Alkyl steht, wobei die Substituenten unabhängig voneinander ausgewählt sein können aus Halogen, Cyano, Nitro, C₁-C₄-Alkoxy, C₁-C₄-Haloalkoxy; und
m für 0 oder 1 steht.

Als Aminoglykol der allgemeinen Formel (3) insbesondere bevorzugte Verbindungen sind Verbindungen der allgemeinen Formel (3), worin
m für 0 steht.

Dieser als Edukt für die erfindungsgemäße Umsetzung gemäß erster Ausführungsform verwendete Aminoglykol kann durch Umsetzung von Acetonoxim mit Ethylencarbonat in Gegenwart von DBU und anschließender Spaltung mit Salzsäure, wie in EP 0 655 437 beschrieben, hergestellt werden.

Aminoglykol kann darüber hinaus vorzugsweise auch durch Umsetzung von Ketonoximen der allgemeinen Formel (4) mit Ethylenoxid in wässriger Lösung und in Gegenwart einer Base hergestellt werden (vgl. US 4,687,849).

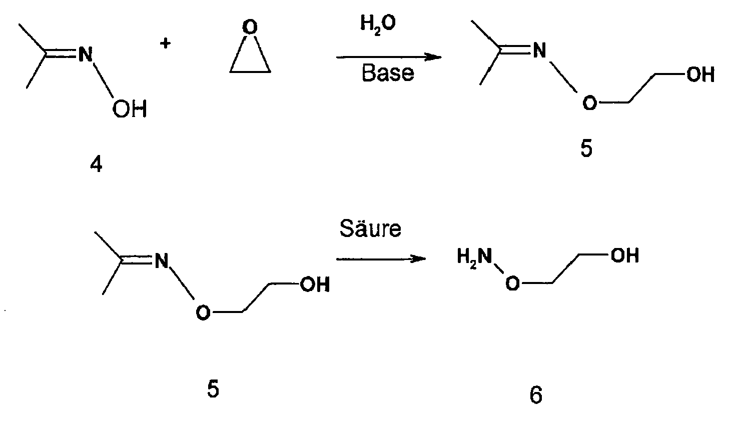

In beiden Fällen wird der Aminoglykol im letzten Verfahrensschritt durch Umsetzung mit einer Säure, üblicherweise Salzsäure (HCl) freigesetzt und liegt somit als saure wässrige Hydrochlorid-Lösung vor. In der erfindungsgemäßen Umsetzung gemäß erster Ausführungsform kann der Aminoglykol in einer entsprechenden sauren wässrigen Lösung eingesetzt werden. Durch das Entfernen von Wasser (z.B. durch Aseotropieren mit Toluol) kann Aminoglykolhydrochlorid jedoch auch als Feststoff isoliert werden und dann in isolierter Form in der erfindungsgemäßen Umsetzung gemäß erster Ausführungsform eingesetzt werden.

Die Umsetzung zwischen dem Nicoitinsäurechlorid oder -ester der allgemeinen Formel (2) und dem Aminoglykol der allgemeinen Formel (3) selbst kann in verschiedenen Lösemitteln durchgeführt werden und unterliegt in dieser Hinsicht keiner besonderen Beschränkung. Entsprechende Beispiele für geeignete Lösemittel sind somit Wasser, Dichlorethan, Dichlormethan, Dimethoxyethan, Diglym, Acetontril, Butyronitril, THF, Dioxan, Ethylacetat, Butylacetat, Dimethylacetamid, Toluol und Chlorbenzol.

In einer besonderen Ausgestaltung der vorliegenden Erfindung wird die Umsetzung gemäß erster Ausführungsform jedoch in einem Zweiphasen-System, bestehend aus Wasser und einem organischen Lösemittel durchgeführt, wobei grundsätzlich die zuvor genannten Lösemittel als organisches Lösemittel in Frage kommen. Besonders bevorzugt ist dabei die Umsetzung in einem Zweiphasen-System aus Ethylacetat/Wasser, Toluol/Wasser, Chlorbenzol/Wasser oder Dichlorethan/Wasser. Die der vorliegenden Erfindung unter anderem zugrunde liegende Erkenntnis, dass die Umsetzung gemäß erster Ausführungsform zwischen dem Nicotinsäurechlorid und dem Aminoglykol tatsächlich in Gegenwart von Wasser durchgeführt werden kann, ist überraschend, da in der Umsetzung als ein Edukt ein Säurechlorid verwendet wird, welches nach der allgemeinen Auffassung in entsprechenden wässrigen Systemen nicht hydrolysestabil ist.

Falls ein entsprechendes Zweiphasen-System verwendet wird, so kann das System darüber hinaus auch mindestens einen Phasentransferkatalysator umfassen.

Von verschiedenen Klassen von Verbindungen ist bekannt, dass sie als Phasentransferkatalysatoren wirken können; beispielsweise sind dies quaternäre Ammoniumverbindungen und Phosphoniumverbindungen. Phasentransferkatalysatoren im Sinne der vorliegenden Erfindung umfassen Tetrabutylammoniumbromid, Tetrabutylammoniumhydroxid, Tetrabutylammoniumhydrogensulfat, TEBA, Tricaprylylmethylammoniumchlorid, wie Aliquat® 336 (hergestellt von Aldrich Chemical Company, Inc., Milwaukee, WI), Dodecylsulfat, Natriumsalz, wie z. B. Natriumlaurylsulfat, Tetrabutylammoniumhydrogensulfat, Hexadecyltributylphosphoniumbromid oder Hexadecyltrimethylammoniumbromid, sind aber nicht hierauf beschränkt. Im Rahmen der vorliegenden Erfindung können als Phasentransfer-Katalysator auch Kronenether, wie zum Beispiel 15-Krone-5, 18-Krone-6 und Benzo-18-Krone-6, verwendet werden.

Darüber hinaus ist es möglich, die Reaktion in einem im Wesentlichen homogenen Gemisch aus Wasser und Lösemitteln durchzuführen, falls das organische Lösemittel mit Wasser mischbar ist.

Die erfindungsgemäße Umsetzung gemäß erster Ausführungsform wird vorzugsweise bei Raumtemperatur durchgeführt. Es können jedoch auch Temperaturen oberhalb von Raumtemperatur, beispielsweise bis 50 °C, und Temperaturen unterhalb Raumtemperatur, beispielsweise bis 0 °C, angewendet werden.

In der ersten Ausführungsform der vorliegenden Erfindung wird der Aminoglykol der allgemeinen Formel (3) vorzugsweise als wässrige Lösung, insbesondere als saure wässrige Lösung, eingesetzt. Der Gewichtsanteil an Aminoglkykol der allgemeinen Formel (3) in der wässrigen Lösung kann dabei in weiteren Bereichen variieren und beträgt vorzugsweise 15 bis 50 Gew.-%, besonders bevorzugt 10 bis 40 Gew.-%, insbesondere 12 bis 35 Gew.-%. Höhere Gewichtsanteile an Aminoglykol sollten auf jeden Fall vermieden werden, da der Aminoglykol bei einer Temperatur von ca. 100°C eine heftige Zersetzungsreaktion zeigt und eventuell schlagempfindlich ist.

Da das gewünschte Nicoitinamid der allgemeinen Formel (1) in der Struktur selbst sowohl ein freies Stickstoffatom an der Amidfunktion sowie eine freie Hydroxylfunktion aufweist, besteht in dem Reaktionssystem grundsätzlich auch das Problem, dass es zu weiteren Reaktionen mit den Verbindungen der allgemeinen Formel (2) kommen kann. Überraschenderweise wurde jedoch in der vorliegenden Erfindung herausgefunden, dass diese Nebenreaktionen im Wesentlichen dadurch unterdrückt werden können, wenn der pH-Wert während der Reaktion im Bereich von vorzugsweise 6 bis 9, besonders bevorzugt 6 bis 8,5, ganz besonders bevorzugt 6 bis 8, gehalten wird. Wenn die erfindungsgemäße Reaktion gemäß erster Ausführungsform bei diesem pH-Wert-Bereich durchgeführt wird, kann eine weitere Acylierung im Wesentlichen vermieden werden. Der pH-Wert kann dabei durch die Zugabe von einer Base wie beispielsweise LiOH, NaOH, NaHCO₃, Na₂CO₃, KOH, K₂CO₃ in dem gewünschten Bereich gehalten werden, wobei die Base auch vor der Zugabe des Säurechlorids vorgelegt werden kann.

Darüber hinaus hat es sich als besonders bevorzugt herausgestellt, wenn zur Durchführung der Umsetzung das Aminoglykolhydrochlorid und NaOH in Wasser, dem Lösemittel oder Mischungen davon vorgelegt werden, und dann das entsprechende Nicotinsäurechlorid bzw. der entsprechende Nicotinsäureester langsam, beispielsweise tropfenweise, zugegeben wird.

Nach Vervollständigung der Reaktion wird das erhaltene Reaktionsprodukt im Allgemeinen so aufgearbeitet, dass der entstandene Niederschlag abfiltriert, gewaschen und im Vakuum getrocknet wird.

Das gewünschte Nicotinamid-Derivat der allgemeinen Formel (1) kann darüber hinaus auch durch eine weitere Ausführungsform der vorliegenden Erfindung gewonnen werden, welche nunmehr näher erläutert wird.

### Zweite Ausführungsform

So ist in einer zweiten Ausführungsform das erfindungsgemäße Verfahren dadurch gekennzeichnet, dass Pyridin-Derivate mit einer Hydroxamsäurefunktion der allgemeinen Formel (7) mit Ethylenoxid der allgemeinen Formel (8) umgesetzt werden.

Erfindungsgemäß wurde nämlich herausgefunden, dass Nicoitinamid-Derivate der allgemeinen Formel (1) durch Umsetzung von Pyridin-Derivate mit einer Hydroxamsäurefunktion der allgemeinen Formel (7) mit Ethylenoxid der Formel (8) unter Ethoxylierung der OH-Gruppe der Hydroxamsäure hergestellt werden können.

Das Ethylenoxid kann dabei ein- bis vierfach substituiert sein, wobei in unten dargestellter Reaktionsgleichung nur eine zweifache Substitution vorgesehen ist.

Das erfindungsgemäße Verfahren gemäß zweiter Ausführungsform kann anhand des folgenden Schemas verdeutlicht werden:

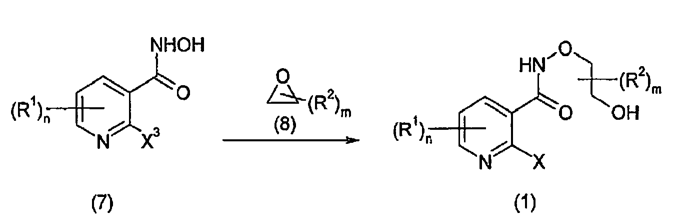

Hinsichtlich der einzelnen Substituenten R¹ und R² sowie den Indizes m und n der Hydroxamsäure der allgemeinen Formel (7) kann auf obige Ausführungen hinsichtlich der Verbindung der allgemeinen Formel (1) verwiesen werden. Darüber hinaus steht X³ für Fluor, Chlor, Brom, lod, SCN, oder S-R^{3"}, wobei R^{3"} für Wasserstoff; gegebenenfalls substituiertes C₁-C₆-Alkyl; gegebenenfalls substituiertes C₃-C₆-Cycoalkyl; -(CH₂)ᵣ-C₆H₅ mit r = 0 bis 6, wobei der Alkylrest - (CH₂)ₘ- gegebenenfalls substituiert sein kann; oder für den Rest 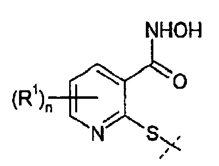 (d.h. Dimerstruktur der allgemeinen Formel (7), wobei der Rest R¹ wie oben stehend definiert sein kann.

Die Synthese von der Hydroxamsäuren der allgemeinen Formel (7) ist aus der US 5,476,936 bekannt.

Die Umsetzung entsprechender Pyridin-Derivate mit Hydroxamsäurefunktion mit Ethylenoxid unter Ausbildung eines gewünschten Nicoitinamid-Derivaten der Formel (1) ist für den Fachmann an sich überraschend, da Ethylenoxid grundsätzlich auch mit der freien Hydroxylfunktion des Nicotinamid-Derivats der allgemeinen Formel (1), also mit dem gewünschten Produkt der erfindungsgemäßen Umsetzung, weiterreagieren kann. Die Bildung entsprechender Nebenprodukte unterbleibt jedoch im Wesentlichen, wenn die erfindungsgemäße Umsetzung gemäß zweiter Ausführungsform angewendet wird.

Darüber hinaus sind aus dem Stand der Technik im Allgemeinen keine Umsetzungen von Hydroxamsäure-Funktionalitäten mit Ethylenoxid bekannt. Die vorliegende Erfindung betrifft daher im Allgemeinen auch ein Verfahren zur Herstellung von Verbindungen der allgemeinen Formel (II) durch Umsetzung von Verbindungen der allgemeinen Formel (I) mit Ethylenoxid der Formel (8): 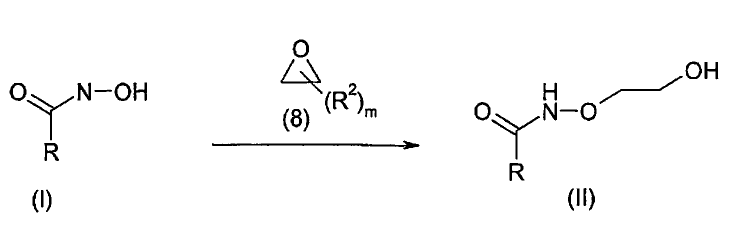 wobei der Rest R einen beliebigen aromatsichen, cyclischen, heteroaromatischen, heterocyclischen oder aliphatischen organischen Rest, vorzugsweise einen aromatischen oder heteroaromatischen Rest, besonders bevorzugt Pyridin, darstellt.

Die erfindungsgemäße Umsetzung gemäß zweiter Ausführungsform wird vorzugsweise in einem Lösemittel durchgeführt, welches ausgewählt wird aus der Gruppe, bestehend aus Wasser und mit Wasser mischbaren Lösemitteln, wie zum Beispiel Aceton, Methanol, Ethanol und Acetonitril. Auch Lösemittelgemische der zuvor genannten organischen Lösemittel mit Wasser können verwendet werden.

Der pH-Wert, bei welchen die erfindungsgemäße Umsetzung gemäß zweiter Ausführungsform durchgeführt wird, liegt vorzugsweise in einem Bereich von 7,5 bis 12,5, besonders bevorzugt 8 bis 12, insbesondere 9 bis 10. Dieser pH-Wert-Bereich hat sich erfindungsgemäß als vorteilhaft herausgestellt, da in diesem Fall eine weitere Ethoxylierung im Wesentlichen vermieden werden kann. Der pH-Wert kann durch die Zugabe einer Base in diesem Bereich gehalten werden.

Die Umsetzung der Hydroxamsäure mit dem Ethylenoxid erfolgt daher vorzugsweise in Gegenwart einer Base. Als Basen können sowohl organische wie auch anorganische Basen verwendet werden. Bevorzugt werden anorganische Basen wie beispielsweise LiOH, NaOH, KOH, Ca(OH)₂, Ba(OH)₂, Li₂CO₃, K₂CO₃, Na₂CO₃, NaH-CO₃, oder organische Basen wie Amine (wie beispielsweise bevorzugt Triethylamin, Diethylisopropylamin), Bu₄NOH, Piperidin, Morpholin, Alkylpyridine, verwendet. Besonders bevorzugt werden anorganische Basen, ganz besonders bevorzugt, LiOH, NaOH und KOH verwendet.

Die Umsetzung wird im Allgemeinen so durchgeführt, dass die Hydroxamsäure in dem entsprechenden Lösemittel oder Wasser vorgelegt wird. Dabei werden vorzugsweise 15 bis 40 gew.-%ige Suspensionen oder Lösungen verwendet. Das Ethylenoxid wird über einen gewissen Zeitraum in die Lösung bzw. Dispersion der Hydroxamsäure eingespeist. Dabei liegt die Temperatur vorzugsweise in einem Bereich von 15 bis 35 °C. Im Allgemeinen werden dabei 1,2 bis 4 Moläquivalente Ethylenoxid, bezogen auf das Pyridin-Derivat mit Hydroxamsäurefunktion, verwendet. Nach vollständiger Zugabe des Ethylenoxids, welche sich über einen Zeitraum von 1 bis 2 Stunden erstrecken kann, kann die Reaktionslösung noch eine gewisse Zeit, beispielsweise für einen Zeitraum von 4 bis 12 Stunden nachgerührt werden.

Die Aufarbeitung erfolgt im Allgemeinen in der Gestalt, dass die Reaktionsmischung auf einen pH-Wert von vorzugsweise 4 bis 7, besonders bevorzugt 4,5 bis 6,5, insbesondere 5 bis 6, eingestellt wird und der Niederschlag abfiltriert wird.

Die Einstellung des pH-Wertes auf den zuvor genannten Bereich erfolgt vorzugsweise durch Zugabe einer Säure. Als Säuren können sowohl organische wie auch anorganische Säuren verwendet werden. Bevorzugt werden anorganische Säuren wie beispielsweise HCl, HBr, HF, H₂SO₄, H₃PO₄ oder organische Säuren wie CF₃COOH, CH₃COOH, p-Toluolsulfonsäure verwendet. Besonders bevorzugt werden anorganische Säuren, ganz besonders bevorzugt HCl und H₂SO₄ verwendet.

Abschließend wird der Niederschlag abfiltriert, mit einem geeigneten Lösemittel gewaschen und abschließend getrocknet.

Für beide Ausführungsformen gilt, dass im Zusammenhang mit der vorliegenden Erfindung substituierte Reste einfach oder mehrfach substituiert sein können, wobei bei Mehrfachsubstitutionen die Substituenten gleich oder verschieden sein können.

Die erfindungsgemäß vorgesehenen Verbindungen können als Mischungen verschiedener möglicher isomerer Formen, insbesondere von Stereoisomeren, wie z.B. E- und Z-, syn und anti, sowie optischen Isomeren, gegebenenfalls aber auch von Tautomeren vorliegen. Es werden sowohl die E- als auch die Z-Isomeren, sowie die optischen Isomeren, beliebige Mischungen dieser Isomeren, sowie die möglichen tautomeren Formen beansprucht.

Weiterhin ist als vorteilhaft zu erwähnen, dass die aus der ersten und zweiten Ausführungsform erhaltenen Produkte ohne zwischenzeitliche Aufreinigung/Isolation für nachfolgende Umsetzungen werden können. Allerdings sind auch Aufreinigungen wie beispielsweise durch Kristallisation, Chromatographie etc. möglich.

Die Erfindung soll anhand der nachfolgenden Ausführungsbeispiele näher erläutert werden, ohne sie jedoch auf diese zu beschränken.

### Erste Ausführungsform

### Beispiel 1:

(2-(Benzylthio)-N-(2-hydroxyethoxy)nicotinamid 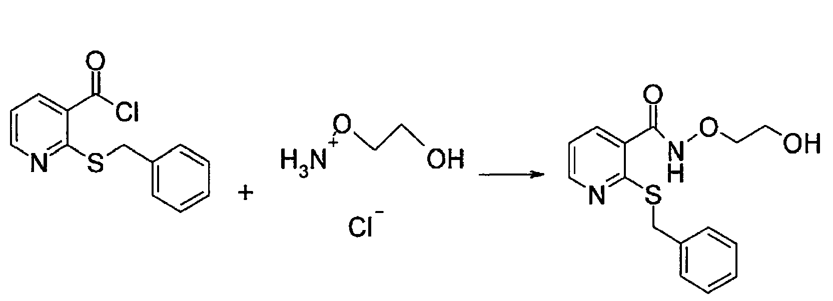 140 g Aminoglykolhydrochlorid wurden als ca. 18 %-ige Lösung in Wasser vorgelegt, und die Lösung wurde mit 20%-igen NaOH-Lösung auf einen pH-Wert von 6,8 - 6.9 eingestellt. 100 ml Essigester wurden zu dem Gemisch zugegeben, und dann wurden 107 g an 2-[(Phenylthio)methyl]nicotinoyl-chloride in Ethylacetat langsam zugegeben. Der pH-Wert wurde während der Reaktion bei 7 mit Hilfe von 20%-iger Na-OH-Lösung stabil gehalten. Der weiße Niederschlag wurde abgesaugt mit Wasser gewaschen und im Vakuumtrockenschrank bei 50 °C getrocknet.

Ausbeute: 114.2 g, 91 % der Theorie, Smp. 141 - 142 °C.
1 H NMR (DMSO d₆) 3.6 (m, 2H), 3.8 (m, 2H), 4.4 (s, 2H), 7.2-7.4 (m, 6H), 7.8 (dd, 1H), 8.5 (dd, 1 H).

### Beispiel 2:

2-Chlor-N-(2-hydroxyethoxy)nicotinamid

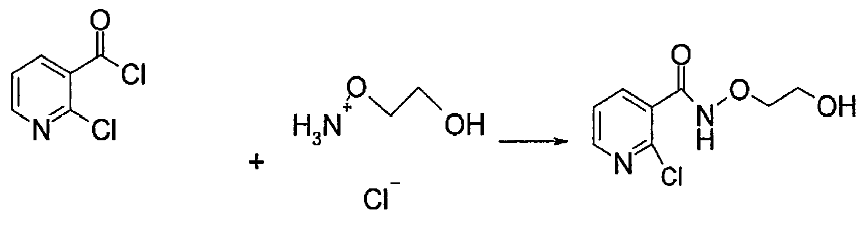

Man arbeitet wie in Beispiel 1 beschrieben, verwendet jedoch 2-Chlornicotinsäurechlorid als Edukt.

Ausbeute 85 %, Öl.
1 H NMR (DMSO d₆) 3.6 (m, 2H), 3.8 (m, 2H), 7.5 (m, 1 H), 7.9 (m, 1 H), 8.5 (m, 1 H).

### Zweite Ausführungsform

### Beispiel 3:

(2-(Benzylthio)-N-(2-hydroxyethoxy)nicotinamid

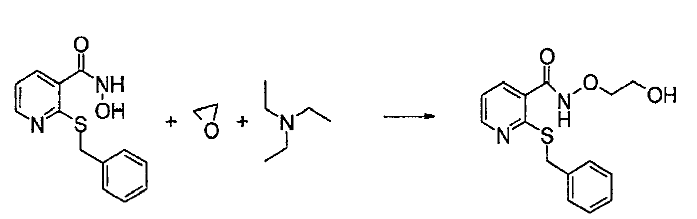

Hydroxamsäure 26 g und Triethylamin 22 g wurden in 400 ml Wasser vorgelegt, und 25 g Ethylenoxid binnen 2 Std. eingeleitet. Das Gemisch wurde danach 8 Std. bei Raumtemperatur nachgerührt. Die Reaktionslösung wurde bei 20 °C mit Essigsäure auf einem pH-Wert von 5 bis 6 eingestellt und der weißer Niederschlag wurde abfiltriert, gewaschen und getrocknet.

Ausbeute 30 g, (84 % der Theorie), Reinheit 95 %. Smp. 140-143°C.

### Beispiel 4:

2-Chlor-N-(2-hydroxyethoxy)nicotinamid

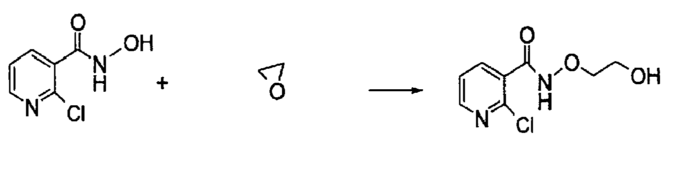

Man arbeitet wie in Beispiel 3 beschrieben, verwendet jedoch 2-Pyridiin-3-hydroxamsäure als Edukt.

Ausbeute 85 %, Öl.
1 H NMR (DMSO d₆) 3.6 (m, 2H), 3.8 (m, 2H), 7.5 (m, 1H), 7.9 (m, 1H), 8.5 (m, 1H).

## Patentansprüche

1. Verbindungen der allgemeinen Formel (1), 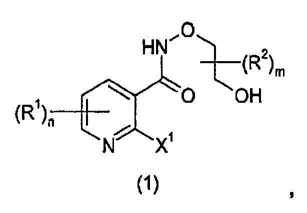 in welchen die Substituenten die folgende Bedeutung aufweisen:
X¹ Fluor, Chlor, Brom, lod, SCN, oder S-R³, wobei
R³ für Wasserstoff;
gegebenenfalls substituiertes C₁-C₆-Alkyl;
gegebenenfalls substituiertes C₃-C₆-Cycoalkyl;
-(CH₂)ᵣ-C₆H₅ mit r = 0 bis 6, wobei der Alkylrest -(CH₂)ᵣ-gegebenenfalls substituiert sein kann; oder 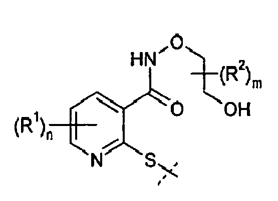 steht;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n eine ganzzahlige Zahl von 0 bis 2;
R² unabhängig voneinander jeweils gegebenenfalls einfach oder mehrfach, gleich oder verschieden substituiertes C₁-C₆-Alkyl, C₂-C₆-Alkenyl, C₂-C₆-Alkinyl, C₃-C₆-Cycloalkyl, wobei die Substituenten unabhängig voneinander ausgewählt sein können aus Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, (C₁-C₆-Alkoxy)carbonyl, (C₁-C₆-Alkyl)carbonyl oder C₃-C₆-Trialkylsilyl; und
m eine ganzzahlige Zahl von 0 bis 4.

2. Verbindungen der allgemeinen Formel (1) nach Anspruch 1, wobei die einzelnen Substituenten die folgende Bedeutung aufweisen:
X¹ Chlor, S-R³, wobei
R³ für gegebenenfalls substituiertes C₁-C₆-Alkyl; gegebenenfalls substituiertes C₃-C₆-Cycoalkyl; -(CH₂)ᵣ-C₆H₅ mit r = 1 bis 4 steht, wobei der Alkylrest -(CH₂)ᵣ- gegebenenfalls substituiert sein kann;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n 0 oder 1;
R² jeweils unabhängig voneinander für gegebenenfalls einfach oder mehrfach, gleich oder verschieden substituiertes C₁-C₄-Alkyl, C₃-C₆-Cycloalkyl, wobei die Substituenten unabhängig voneinander ausgewählt sein können aus Halogen, Cyano, Nitro, Hydroxy, C₁-C₄-Alkoxy, C₁-C₄-Haloalkoxy, C₁-C₄-Alkylthio, C₁-C₄-Alkylsulfinyl und C₁-C₄-Alkylsulfonyl; und
m eine ganzzahlige Zahl von 0 bis 2.

3. Verbindungen der allgemeinen Formel (1) nach Anspruch 1 oder 2, wobei die einzelnen Substituenten die folgende Bedeutung aufweisen:
X¹ Chlor, S-R³, wobei
R³ für gegebenenfalls substituiertes C₁-C₆-Alkyl; gegebenenfalls substituiertes C₃-C₆-Cycoalkyl; -(CH₂)ᵣ-C₆H₅ mit r = 1 oder 2 steht, wobei der Alkylrest -(CH₂)ᵣ- gegebenenfalls substituiert sein kann;
R¹ Halogen; Cyano; Thiocyanato; oder für jeweils gegebenenfalls durch Halogen substituiertes Alkyl, Alkenyl, Alkinyl, Alkoxy, Alkylthio, Alkylsulfinyl, Alkylsulfonyl, Alkylamino, Alkylcarbonyl, Alkoxycarbonyl, Alkylaminocarbonyl, Aryl, Heteroaryl, Cycloalkyl und Heterocyclyl, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 oder 10 C-Atome enthalten können;
n 0 oder 1;
R² gegebenenfalls einfach oder mehrfach, gleich oder verschieden substituiertes C₁-C₄-Alkyl, wobei die Substituenten unabhängig voneinander ausgewählt sein können aus Halogen, Cyano, Nitro, C₁-C₄-Alkoxy, C₁-C₄-Haloalkoxy; und
m 0 oder 1.

4. Verbindungen der allgemeinen Formel (1) nach einem der Ansprüche 1 bis 3, **dadurch gekennzeichnet, dass** die einzelnen Substituenten die folgende Bedeutung aufweisen::
X¹ S-CH₂-C₆H₅;
n 0; und
m 0.

5. Verfahren zur Herstellung einer Verbindung der allgemeinen Formel (1), nach einem der Ansprüche 1 bis 4, **dadurch gekennzeichnet, dass** Nicotinsäurechloride oder -ester der allgemeinen Formel (2) mit Aminoglykol der allgemeinen Formel (3) umgesetzt werden, wobei der Substituent Y für Chlor oder gegebenenfalls substituiertes -O(C₁-C₆-Alkyl) steht, die Substituenten R¹, R² und X¹ sowie die Indizes m und n dieselbe Bedeutung wie in einem der Ansprüche 1 bis 4 aufweisen und X² für Fluor, Chlor, Brom, lod, SCN, oder S-R^{3'} steht, wobei
R^{3'} für Wasserstoff;
gegebenenfalls substituiertes C₁-C₆-Alkyl;
gegebenenfalls substituiertes C₃-C₆-Cycoalkyl;
-(CH₂)ᵣ-C₆H₅ mit r = 0 bis 6, wobei der Alkylrest -(CH₂)ᵣ-gegebenenfalls substituiert sein kann; oder den Rest 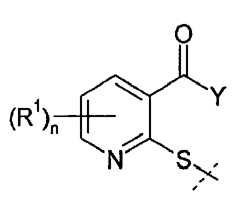 mit Y gleich Chlor oder gegebenenfalls substituiertes -O(C₁-C₆-Alkyl) steht und wobei R¹ und n in dem Rest eine Bedeutung wie in einem der Ansprüche 1 bis 4 aufweisen:

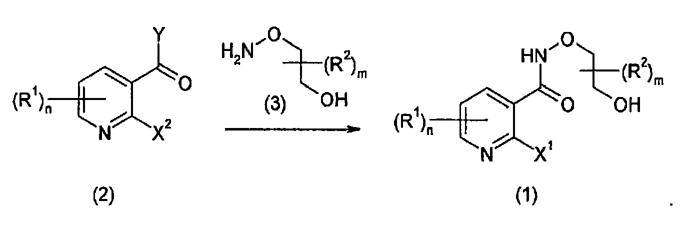

6. Verfahren nach Anspruch 5, **dadurch gekennzeichnet, dass** die Umsetzung in einem Zweiphasen-System aus Ethylacetat/Wasser, Toluol/Wasser, Chlorbenzol/Wasser oder Dichlorethan/Wasser als Lösemittelsystem durchgeführt wird.

7. Verfahren nach Anspruch 6, **dadurch gekennzeichnet, dass** das Zweiphasen-System darüber hinaus auch mindestens einen Phasentransferkatalysator umfasst.

8. Verfahren nach einem der Ansprüche 5 bis 7, **dadurch gekennzeichnet, dass** der Aminoglykol der allgemeinen Formel (3) als wässrige Lösung, vorzugsweise als saure wässrige Lösung, eingesetzt wird.

9. Verfahren nach Anspruch 8, **dadurch gekennzeichnet, dass** der Aminoglykol der allgemeinen Formel (3) als wässrige Lösung, vorzugsweise als Hydrochloridsalz-Lösung, eingesetzt wird.

10. Verfahren zur Herstellung von Verbindungen der allgemeinen Formel (II) 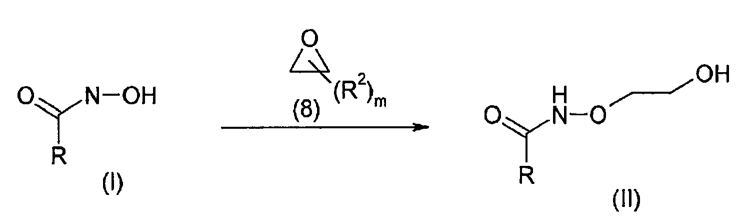 durch Umsetzung von Hydroxamsäureresten der allgemeinen Formel (I) mit Ethylenoxid der Formel (8), wobei der Rest R einen beliebigen aromatsichen, cyclischen, heteroaromatischen, heterocyclischen oder aliphatischen organischen Rest darstellt.

11. Verfahren nach Anspruch 10 zur Herstellung von Nicotinamiden der allgemeinen Formel (1), **dadurch gekennzeichnet, dass** Hydroxamsäurederivate der allgemeinen Formel (7) mit Ethylenoxid der Formel (8) unter Ringöffnung des Ethylenoxids umgesetzt werden 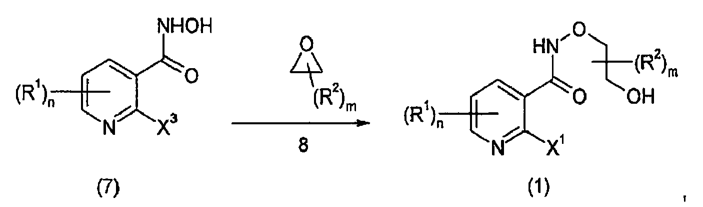 wobei die Substituenten R¹, R² und X¹ sowie die Indizes n und m dieselbe Bedeutung wie in einem der Ansprüche 1 bis 4 aufweisen und X³ für Fluor, Chlor, Brom, Iod, SCN, oder S-R^{3"}, wobei R^{3"} für Wasserstoff; gegebenenfalls substituiertes C₁-C₆-Alkyl; gegebenenfalls substituiertes C₃-C₆-Cycoalkyl; - (CH₂)ᵣ-C₆H₅ mit r = 0 bis 6, wobei der Alkylrest -(CH₂)ₘ- gegebenenfalls substituiert sein kann; oder für den Rest 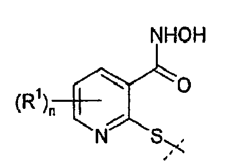 steht, wobei R¹ und der Index n in dem Rest wie oben stehend definiert sein kann.

12. Verfahren nach Anspruch 11, **dadurch gekennzeichnet, dass** die Umsetzung ei einem pH-Wert in einem Bereich von 8 bis 13 durchgeführt wird.

13. Verfahren nach Anspruch 11 oder 12, **dadurch gekennzeichnet, dass** die Umsetzung in einem Lösemittel durchgeführt wird, welches ausgewählt wird aus der Gruppe, bestehend aus Wasser und mit Wasser mischbaren Lösemitteln, insbesondere Aceton, Methanol, Ethanol, N,N-Dimethylformamid und Acetonitril.
